# FASCICULE DE BREVET EUROPEEN

(11) **EP 0 767 375 B1**
(45) Date de publication et mention de la délivrance du brevet: **06.03.2002**
(21) Numéro de dépôt: 96402071.3
(22) Date de dépôt: 27.09.1996
(51) Int. Cl.: G01N 33/24, G01N 31/12

(54) **Méthode et dispositif pour déterminer des caractéristiques pétrolières de sédiments géologiques**
Verfahren und Vorrichtung zur Bestimmung der petrologischen Eigenschaften von Schichtgesteinen
Method and apparatus for determining the petrological properties of sedimentary rock

(30) Priorité: 05.10.1995 FR 9512001
(43) Date de publication de la demande: 09.04.1997
(73) Titulaire: INSTITUT FRANCAIS DU PETROLE, 92500 Rueil Malmaison (FR)
(72) Inventeur: Espitalie, Jean, 78110 Le Vesinet (FR); Marquis, François, 95680 Montlignon (FR)

(56) Documents cités:
- EP-A- 0 269 511
- EP-A- 0 584 377
- FR-A- 2 472 754
- GB-A- 2 173 305
- SOVIET PATENT ABSTRACTS Section Ch, Week 9517 Derwent Publications Ltd., London, GB; Class H01, AN 95-129356 XP002007153 & SU-A-1 336 710 (GEOLOGICAL SURVEY RES INST) , 15 Août 1994

## Description

La présente invention concerne une méthode et un dispositif perfectionné pour déterminer certaines au moins des caractéristiques pétrolières d'un sédiment géologique, telles que par exemple, mais non exclusivement, la possibilité de produire du pétrole immédiatement ou dans l'avenir, c'est-à-dire d'être une bonne roche-mère, ou encore d'être une roche réservoir contenant des hydrocarbures.

La présente invention concerne un perfectionnement à la technique ROCKEVAL (marque déposée par l'Institut Français du Pétrole), ainsi qu'une optimisation du dispositif de mesure pour appliquer la méthode. La technique ROCKEVAL antérieure est décrite dans les documents FR 2339173 et FR 2472754.

La méthode et les dispositifs antérieurs ne permettent pas de mesurer le CO₂ d'une manière continue pendant les cycles de chauffe: pyrolyse ou oxydation. De plus, il n'est pas possible de mesurer le CO qui se dégage simultanément au CO₂. Or, cette analyse du CO, lequel est produit uniquement par le craquage de la matière organique, est notamment indispensable pour une meilleure détermination du carbone organique total. Cette analyse permet, par exemple, une meilleure précision de l'indice d'oxygène IO en incluant au CO₂ le CO ainsi analysé. Egalement, la simple interprétation en conjugaison des courbes continues de CO et CO₂ permet de différencier l'oxygène organique de l'oxygène minéral.

Ainsi la présente invention concerne une méthode pour permettre la détermination d'au moins une caractéristique pétrolière d'un échantillon de sédiment géologique, dans laquelle l'échantillon est chauffé en atmosphère non oxydante, sa température étant élevée successivement jusqu'à une première une seconde valeur, ladite première valeur inférieure à 200°C étant atteinte très rapidement puis maintenue sensiblement constante pendant un certain temps, ladite seconde valeur comprise entre 600°C et 850°C étant atteinte selon un gradient de température compris entre 0,2 et 50°C/min, à partir de ladite première valeur. Selon l'invention, on mesure en continu, à chaque instant de la période de chauffe dudit échantillon, la quantité de CO₂ contenu par l'effluent résultant de ladite chauffe, on peut tracer une courbe représentative de ladite quantité de CO₂ et on peut différencier le CO₂ d'origine organique du CO₂ d'origine minéral à partir des mesures continues et, notamment à partir de la forme de la courbe.

On peut mesurer en continu, à chaque instant de la période de chauffe dudit échantillon, la quantité de CO contenu par l'effluent résultant de ladite chauffe, et on peut tracer une courbe représentative de ladite quantité de CO.

On peut différencier l'oxygène organique de l'oxygène minéral par l'interprétation conjuguée des mesures continues et notamment au moyen des courbes de CO₂ et de CO.

Selon la méthode, on place les résidus de ladite chauffe en atmosphère non oxydante dans un autre four où ils sont chauffés en atmosphère oxydante, la chauffe en atmosphère oxydante peut être programmée en température de façon à passer d'une température d'environ 400°C à une température finale d'environ 850°C par un gradient de température compris entre 10 et 30°C/min. On peut mesurer en continu, à chaque instant de la période de chauffe desdits résidus, la quantité de CO₂ contenu par l'effluent résultant de ladite chauffe. On peut tracer une courbe représentative de ladite quantité de CO₂ et on peut différencier le CO₂ d'origine organique du CO₂ d'origine minéral à partir des mesures continues et notamment de la forme de ladite courbe.

On peut mesurer en continu, à chaque instant de la période de chauffe en atmosphère oxydante desdits résidus, la quantité de CO contenu par l'effluent résultant de ladite chauffe et on peut tracer une courbe représentative de ladite quantité de CO.

On peut calculer la quantité de carbone organique total contenue dans ledit échantillon à partir des mesures continues et notamment des courbes de CO₂ et CO obtenues à la suite des cycles de chauffe en atmosphère non oxydante et en atmosphère oxydante.

L'invention concerne également un dispositif pour permettre la détermination d'au moins une caractéristique pétrolière d'un échantillon de sédiment géologique placé dans une nacelle, le dispositif comportant un premier moyen de chauffe dudit échantillon sous atmosphère non oxydante, des moyens de mesure de la quantité des produits hydrocarbonés dégagés à la suite de l'introduction de l'échantillon dans ledit premier moyen de chauffe, un second moyen de chauffe sous atmosphère oxydante, des moyens de mesure de la quantité de CO₂ contenue dans les effluents évacués des deux moyens de chauffe. Les moyens de mesure du CO₂ comportent une cellule de mesure en continu du CO₂ à chaque instant du cycle de chauffe du premier et du second moyen de chauffe et en ce qu'il comporte des moyens de mesure de la quantité de CO contenue dans les effluents évacués par les deux moyens de chauffe.

Les premier et second moyen de chauffe peuvent comporter chacun un four identique et des moyens de programmation de la température de chauffe des deux fours.

Les fours peuvent comporter un corps sensiblement cylindrique et une cavité intérieure destinée à recevoir la nacelle contenant l'échantillon, ledit corps ayant sensiblement trois parties, une partie supérieure comportant un conduit intérieur en communication avec ladite cavité et avec les moyens de mesure de la quantité des produits hydrocarbonés, une partie inférieure comportant l'ouverture de ladite cavité, une partie centrale dans laquelle sera placée la nacelle, les trois parties peuvent être entourées d'un élément électrique de chauffe bobiné hélicoïdalement à pas jointifs pour les deux parties supérieure et inférieure et à pas sensiblement double pour la partie centrale.

Le dispositif peut comporter des moyens d'introduction de la nacelle dans lesdits moyens de chauffe comportant des moyens de mesure de la température au niveau de la nacelle et des moyens de circulation d'un fluide non oxydant, par exemple de l'azote ou de l'hélium, ou d'un fluide oxydant, par exemple de l'air ou de l'oxygène.

Les moyens de chauffe peuvent comporter chacun un moyen de mesure de la température placé dans la paroi de la partie centrale dudit corps, sensiblement au niveau de la nacelle.

Le dispositif peut comporter des moyens de régulation et de programmation de la température des moyens de chauffe pilotés par lesdits moyens de mesure de la température desdits moyens d'introduction, et lesdits moyens de régulation et de programmation peuvent être pilotés par lesdits moyens de mesure de la température placés dans la paroi de la partie centrale dudit corps lorsque la nacelle est hors des moyens de chauffe, ceux-ci étant alors ouverts.

Le moyen de chauffe en atmosphère non oxydante peut comporter un moyen de balayage de la cavité intérieure du corps de chauffe par un fluide non oxydant lorsque ledit corps est ouvert.

Le dispositif peut comporter des moyens de déplacement de la nacelle entre les deux moyens de chauffe et un magasin, comportant un bras équipé d'une pince à nacelle, ledit bras étant commandé en rotation pour déplacer une nacelle vers une des trois positions et en hauteur pour saisir ou déposer la nacelle, et le magasin peut avoir la forme d'un plateau rotatif sur lequel différentes nacelles sont disposées en cercle.

Le dispositif peut comporter un premier couple de cellules de mesure en continu du CO₂ et du CO contenus par l'effluent dégagé par le premier moyen de chauffe et un deuxième couple de cellules de mesure en continu du CO₂ et du CO contenus par l'effluent dégagé par le second moyen de chauffe.

Le dispositif peut comporter des moyens électroniques d'interface entre les moyens de mesures, de commande de vannes ou de distributeurs, de régulation de la température, de déplacement des nacelles et un calculateur comportant des moyens de mémorisation et d'affichage.

L'invention concerne un système d'évaluation pétrolière à partir d'échantillon de sédiment géologique. le système comporte:
- un dispositif tel que décrit ci-dessus,
- un calculateur de type PC interfacé avec ledit dispositif et comportant des périphériques de visualisation, d'impression et d'introduction des paramètres de fonctionnement,

le calculateur comportant un logiciel multitâche comportant un menu proposant plusieurs cycles préprogrammés d'analyses pouvant être effectués à l'aide dudit dispositif, parmi lesquels: le cycle d'analyse des roches mères, le cycle d'analyse des kérogènes ou charbons, le cycle d'analyse pour déterminer les paramètres cinétiques de la dégradation d'une matière organique, le cycle d'analyse de roches réservoirs, le cycle d'analyse préparative à la maturation.

Le logiciel multitâche peut permettre l'affichage de mesures ou de courbes caractéristiques en temps réel, ledit logiciel comportant des moyens de modification du cycle choisi en cours d'accomplissement en fonction des mesures ou courbes caractéristiques obtenues.

La présente invention sera mieux comprise et ses avantages apparaîtront à la lecture de la description qui suit, illustrée par les figures annexées parmi lesquelles:
- la figure 1 représente le schéma de principe du dispositif de mesure,
- la figure 2 montre le principe des organes de chauffe,
- les figures 3A et 3B montrent les enregistrements types réalisés avec le présent dispositif,
- la figure 4 représente schématiquement l'ensemble d'évaluation pétrolière.

Le principe de la méthode est exposé ci-après. Un échantillon de roche ou de matière organique concentrée est soumis à une loi de chauffe programmée, d'abord sous atmosphère inerte, puis sous gaz oxydant tel que chaque dégagement de produit analysé corresponde à la température réelle de l'échantillon à l'instant de la production. Les effluents sont analysés en continu par un détecteur à ionisation de flamme (FID) pour les hydrocarbures dégagés, et par un dispositif à infrarouges (IR) pour le CO₂ et le CO produits par le craquage de la matière organique ou la décomposition des carbonates.

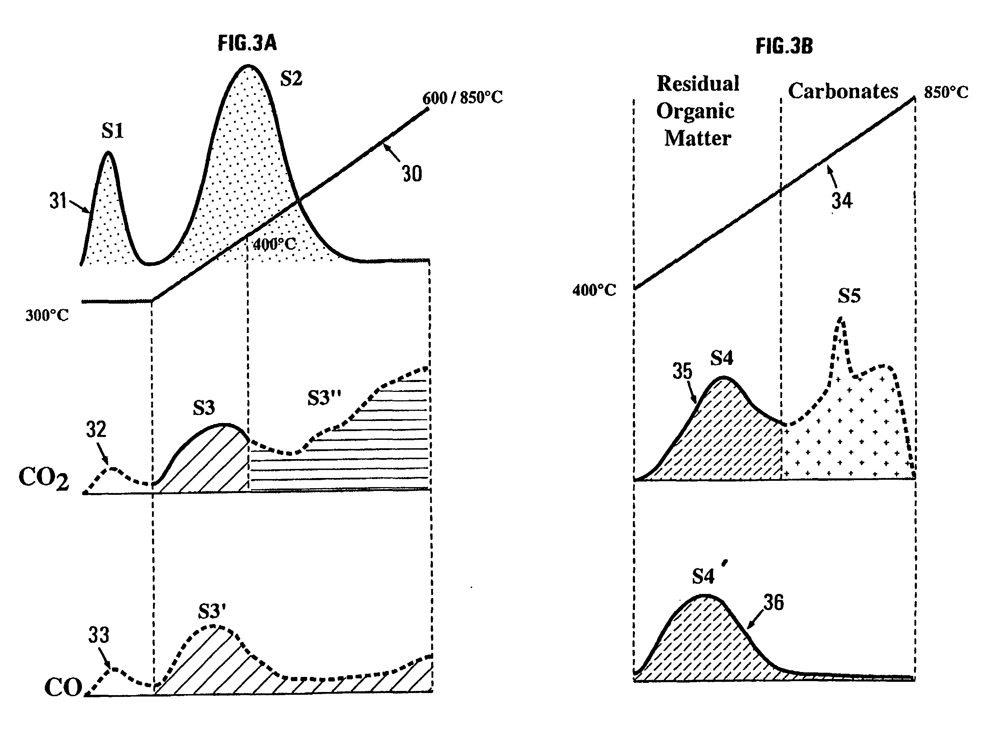

Les différentes courbes sont ensuite intégrées et interprétées pour obtenir les premiers résultats de l'analyse:
- La quantité d'hydrocarbures libres (pic S₁, figure 3A).
- Le potentiel pétrolier (pic S₂, figure 3A).
- Le Tmax (température maximale au sommet du pic de pyrolyse).
- Les quantités de CO₂ et de CO (pics S₃ et S3', figure 3A) obtenues au cours du craquage de la matière organique dans des tranches de températures déterminées. Les sommets des courbes sont donnés en température vraie grâce au dispositif de mesure en continu.
- Le carbone résiduel est déterminé par les quantités de CO₂ et de CO obtenues au cours de l'oxydation du résidu de pyrolyse (pics S4 et S4', figure 3B). Les sommets de chaque courbe sont également déterminés en température vraie.
- Le carbone minéral est obtenu généralement par le pic S5 et S3" correspondant à la décomposition de la calcite et de la dolomie. Les maxima du dégagement de CO₂ sont notés pour déterminer la composition du carbonate. Les minima sont déterminés pour décomposer le pic S5 en carbonates différents.

De ces éléments de base seront déduits les paramètres classiques de l'analyse tels que IP (index de production), IH, IO, et tous autres rapports nécessaires et dérivés des nouvelles fonctions.

Le dispositif selon l'invention est composé d'un automate qui effectue les mesures et d'un PC qui pilote l'automate, sert d'interface avec d'autres calculateurs, gère les analyses, permet la visualisation des résultats en temps réel et qui utilise des logiciels de contrôle et de tests.

Le dispositif de mesure comporte deux micro fours, un passeur d'échantillons qui les alimente en nacelles et un système d'analyse composé par un détecteur à ionisation de flamme (FID) et de deux cellules à infrarouge (IR). Ces éléments sont connectés à une électronique et à un circuit de fluides gérés par le PC et par le software de l'automate.

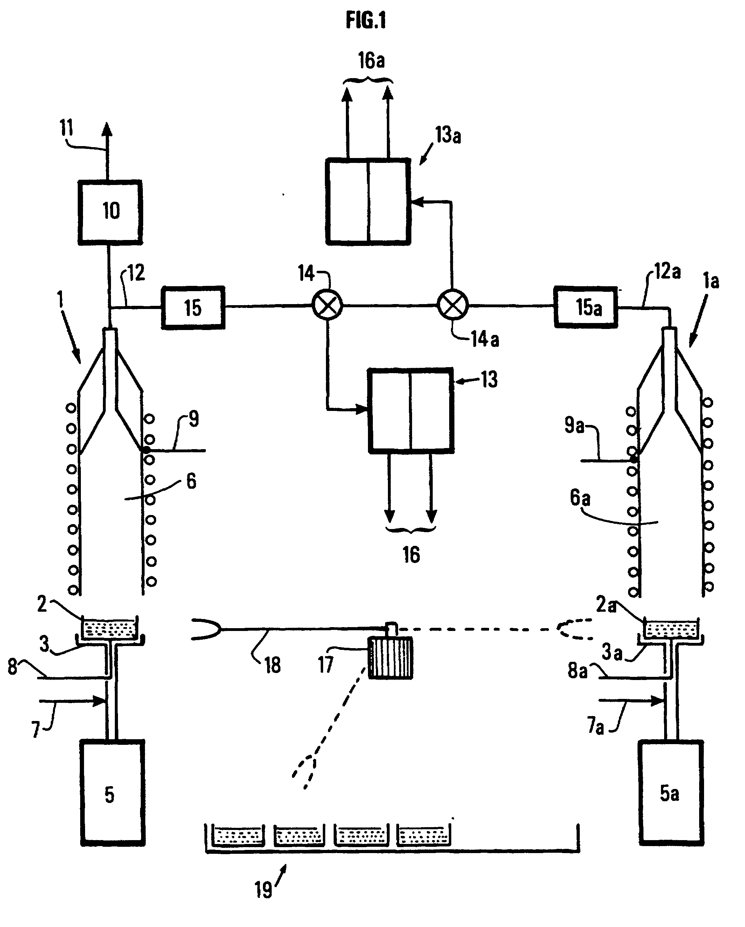

La figure 1 décrit le dispositif perfectionné de pyrolyse et d'oxydation selon la présente invention. La référence 1 désigne l'ensemble de chauffe adapté à la pyrolyse de l'échantillon 2 placé dans une nacelle 3 portée par un piston 4. Des moyens de déplacements 5 de la nacelle introduisent l'échantillon dans l'espace intérieur 6 du four. Les moyens de déplacements peuvent être des vérins à commande pneumatique, hydraulique ou électrique. La référence 7 schématise le conduit d'amenée du fluide vecteur de balayage des produits pyrolysés dans le four. Ce fluide (azote ou hélium) balaye l'échantillon en passant par le piston. Des moyens de distribution (non représentés) conduisent le fluide vecteur à la partie supérieure du four pour effectuer une purge en rétro-balayage de l'intérieur du four lors du recul du piston, par exemple en fin de pyrolyse pour transférer l'échantillon et/ou pour charger un autre échantillon. En effet, l'influence de l'oxygène sur les dépôts organiques des parois du four de pyrolyse peut générer des composés oxygénés CO₂ et CO qui sont gênants pour l'analyse.

Une sonde de température 8 mesure la température au niveau du fond de la nacelle, donc très proche de l'échantillon. Une autre sonde de température 9 a son point de mesure dans la paroi du four, au niveau de la position haute de la nacelle, position correspondant au point de chauffe optimal. La programmation en température du four est effectuée de préférence à l'aide de la sonde 8, ce qui permet un bon contrôle et une bonne connaissance de la température de pyrolyse de l'échantillon. La sonde de température 9 est utilisée pour contrôler la température du four 1 lorsque le four est ouvert et que le piston 4 est descendu pour extraire la nacelle 3 et la remplacer par une autre. Ainsi, la température du four 1 peut être maintenue à une valeur proche de la valeur déterminée pour la pyrolyse suivante ce qui permet d'éviter trop de perte thermique.

L'ensemble de chauffe 1A est identique en tout point à l'ensemble de chauffe 1. Cet ensemble 1A est dévolu à l'opération d'oxydation d'un échantillon, généralement après pyrolyse. Les éléments identiques sont indexés "A". Il faut noter que le fluide injecté par le conduit 7A est dans ce cas de l'air.

Les ensembles de chauffe 1 et 1A ont tous les deux des moyens de régulation de la température qui permettent une programmation du gradient de température, laquelle pouvant atteindre et même dépasser 850°C.

La référence 10 désigne le détecteur FID à flamme ionisante délivrant un signal S représentatif des quantités de produits hydrocarbonés dégagés de l'échantillon en cours de chauffe. La flèche 11 symbolise le transfert du signal S vers les moyens de digitalisation. Le détecteur à ionisation de flamme FID doit tenir aux températures élevées d'où la nécessité pour lui de contenir des joints supportant ces conditions sans fluer ni désorber des produits pouvant faire dévier la ligne de base.

Sa linéarité et sa sensibilité associées à une dérive de la ligne de base très faible sont les gages d'une analyse de grande précision des hydrocarbures.

Le signal analogique sera digitalisé et lissé avec le maximum de points qui est fonction de la vitesse de programmation.

Le conduit 12 amène une partie du flux à des moyens 13 d'analyse en continu des quantités de CO₂ et de CO produits par pyrolyse de l'échantillon. En sortie du four de pyrolyse, la division du flux est chauffée au moins à 360°C pour éviter les condensations de produits lourds.

Le conduit 12A amène au moins une partie du flux d'oxydation vers des moyens 13A d'analyse en continu des quantités de CO et CO₂ produits.

Des moyens de distribution 14 et 14A permettent d'utiliser seulement l'un ou l'autre des moyens d'analyse du CO et CO₂ pour le flux venant de la pyrolyse ou de l'oxydation. De préférence, pour des raison de gain de temps opérationnel, les moyens 13A et 13 seront affectés à un seul moyens de chauffe. Les moyens d'analyse en continu sont par exemple des détecteurs à infrarouge.

Les cellules IR, étant spécifiques d'un gaz, peuvent mesurer en continu les concentrations de CO₂ et de CO dans les effluents au cours de la pyrolyse et de l'oxydation. Elles donnent accès à de nouveaux renseignements tels que la présence et la quantité de différents carbonates, les températures des maxima de dégagement, la forme des pics, la coupure entre le carbone minéral et le carbone organique et la répartition de chaque composé oxygéné dans les différentes réactions de craquage de la matière organique.

La longueur des cellules des détecteurs dépend de la sensibilité maximum demandée donc de la concentration minimum qui doit être mesurée. Elle est fonction, des quantités de CO ou CO₂ produites par l'échantillon (donc de sa masse), de la durée de l'analyse (donc des conditions de chauffage) et du débit du gaz vecteur qui est un facteur diluant.
* La cellule analysant le CO₂ mesure des concentrations maxima de 2% pour un débit variant de 50 à 200 ml. Cette plage est linéarisée sur quatre gammes à changement automatique:
   gamme 1 0 à 2% de CO₂
   gamme 2 : 0 à 1 % de CO₂
   gamme 3 : 0 à 0.5% de CO₂
   gamme 4 0 à 0.25% de CO₂
* La cellule analysant le CO mesure des concentrations maxima de 1% dans les mêmes conditions que la cellule CO₂. Les 4 gammes sont:
   gamme 1 : 0 à 1 % de CO
   gamme 2 : 0 à 0.5% de CO
   gamme 3 : 0 à 0.25% de CO
   gamme 4 : 0 à 0.125% de CO

Les signaux récupérés sur les IR sont remis en forme pour obtenir des courbes à la même atténuation, digitalisés comme pour le signal FID.

Le dispositif comporte également des moyens de purification 15 et 15A des flux.

Les flèches 16 et 16A symbolisent les transferts des mesures vers les moyens électroniques de digitalisation.

De plus, le dispositif comporte un passeur 17 d'échantillons dont le bras 18 est adapté à déplacer la nacelle d'un échantillon entre trois positions possibles: le four de pyrolyse, le four d'oxydation, le magasin 19.

La mécanique du passeur est simplifiée de façon à ce que les déplacements puissent être effectués par des moteurs électriques pas à pas. Ainsi, toutes les possibilités de commande sont offertes et elles ne dépendent que du logiciel de fonctionnement. Il sera possible, par exemple, de charger les nacelles uniquement dans le four d'oxydation pour des études particulières. Une autre application possible sera de traiter thermiquement des échantillons dans un four et de les récupérer ensuite sur le support de nacelles ou magasin (19) pour les analyser selon le cycle Rock-Eval désiré.

Le support des nacelles n'est pas linéaire mais circulaire: il prend la forme d'un carrousel qui occupe moins de place et qui permet d'accéder plus rapidement à la nacelle désirée par une marche avant ou arrière du chargeur. A chaque emplacement est assigné un numéro qui permet de programmer le passage des échantillons non seulement dans l'ordre chronologique du positionnement des nacelles sur le passeur mais aussi, soit en fonction des différents types de cycles ou d'analyses, soit en fonction des priorités d'analyse.

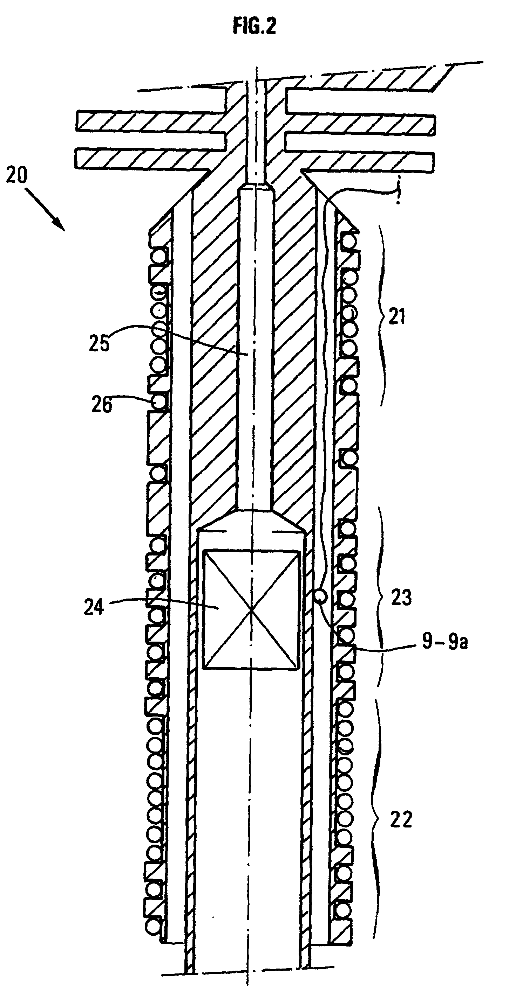

La figure 2 montre la structure particulière des fours de pyrolyse et d'oxydation. Une optimisation des organes chauffants est nécessaire pour pouvoir obtenir des températures au moins supérieures à 850°C et une température initiale d'au moins 100°C, une bonne linéarité des gradients thermiques imposés, une économie d'énergie et une température à l'échantillon très proche de la valeur de consigne.

Le four 20 a une forme tubulaire et comporte trois parties principales: deux parties d'extrémités 21 et 22, et une partie centrale 23 au niveau de laquelle est placé l'échantillon en position d'essai, selon l'emplacement schématisé et référencé 24.

L'espace intérieur du tube constituant le four 20 a un diamètre réduit 25 au niveau de l'extrémité aval du four par rapport au sens de circulation du flux vecteur des effluents de pyrolyse ou d'oxydation. Le bobinage de la résistance électrique de chauffe 26 est optimisé de la façon suivante: la résistance est principalement bobinée à pas jointifs dans les deux parties d'extrémité 21 et 22, alors que dans la partie centrale 23, le pas de bobinage est plus grand, par exemple doublé. La résistance électrique chauffe le corps du four par contact, le contact pouvant être amélioré en noyant la résistance dans un alliage métallique. Le point de mesure de la température par le thermocouple 9 ou 9A est placé au plus près de la paroi intérieure du four, au niveau de l'emplacement 24.

Les fours ainsi structurés ont les caractéristiques avantageuses suivantes:
* Les températures initiales Ti et finales Tf varient entre 100°C et 900°C.
* Le programme de chauffage est à multipentes. Il est possible d'introduire un ou plusieurs segments de programme entre Ti et Tf pouvant être des isothermes de durées variables ou des rampes dont les vitesses varient de 0.2°C/min à 50°C/min.
* La référence en cours d'analyse étant la température, des tops correspondant à des températures bien définies sont utilisés pour effectuer des opérations de commutation ou de connexion. La courbe de montée en température sera associée à toutes les courbes d'analyse.
* L'inertie thermique et la puissance requise étant faibles, cela donne la possibilité de diminuer la température initiale T1 et de faciliter le refroidissement des fours pour un retour rapide à la température initiale.

On pourra donc effectuer, à basse température (environ à 100°C), la volatilisation des hydrocarbures libres les plus légers (inférieur à C5-C6) contenus dans une roche. Puis on provoquera une séparation des composants selon leur point d'ébullition en effectuant une programmation de température adéquate. Cette technique peut être appliquée, soit à l'étude des roches mères, soit à celle des imprégnations de réservoirs.

La possibilité d'effectuer une pyrolyse jusqu'à environ 850°C, permet d'obtenir, particulièrement dans le cas de certains échantillons, la volatilisation ou la dégradation complète de la matière organique résiduelle contenue dans les roches mères ou dans les roches réservoirs (hydrocarbures de C6 à C40 et les résines et asphaltènes). Le potentiel pétrolier de l'échantillon se trouve augmenté, dans certains cas, d'une quantité non négligeable par rapport à une pyrolyse classique à 600 °C ce qui donne un IH plus représentatif.

De plus, les paramètres cinétiques des matières organiques d'origine terrestre, déterminés à l'aide de la pyrolyse, sont plus précis quand les courbes de pyrolyse d'ajustement reviennent à la ligne de base (cas des courbes issues de programmations à vitesses rapides).

La pyrolyse haute température est aussi utile pour déterminer les fortes maturités des roches mères à l'aide de l'échelle des Tmax. Une pyrolyse classique jusqu'à 650°C donne, en effet, dans certains cas de roches matures, un pic S₂ qui ne représente que le début du craquage sans atteindre le maximum du pic. Avec la pyrolyse à haute température on accède par contre à des Tmax supérieurs à environ 587°C grâce à une pyrolyse complète de l'échantillon.

Une température d'oxydation en programmation de température pouvant atteindre 850°C, au lieu du chauffage isotherme à 600°C utilisés actuellement, permet de brûler les cokes les plus résistants. Il est alors possible de mesurer tout le carbone résiduel, y compris dans le cas, difficile des roches mères très matures (cas des charbons très évolués). Les valeurs de TOC sont alors plus justes, et tout à fait comparables aux valeurs obtenues par combustion à 1500°C dans un appareil du type LECO ou par la méthode de l'analyse élémentaire classique.

L'oxydation haute température est nécessaire pour décomposer les carbonates tels que la calcite et la dolomite qui ne peut se faire qu'à des températures supérieures à 600°C. On accède ainsi au carbone minéral total des roches (en prenant en compte la sidérite contenue dans la courbe S₃) et à différentes variétés de carbonates.

Les figures 3A et 3B montrent des mesures types effectuées avec le dispositif décrit figure 1.

Les graphes de la figure 3A concernent les mesures et enregistrements au cours de la pyrolyse d'un échantillon. Les graphes de la figure 3B concernent les mesures et enregistrements au cours de l'oxydation de l'échantillon, après pyrolyse.

Sur la figure 3A, la courbe 30 représente la température à laquelle a été soumis l'échantillon. Dans l'exemple donné ici, on effectue un palier isotherme à 300°C suivi d'une augmentation de température selon un gradient déterminé pour atteindre en fin de pyrolyse une température maximale de 850°C.

La courbe 31 représente le signal enregistré par le détecteur FID. Elle comporte un premier pic S1 correspondant à la volatilisation des hydrocarbures libres pendant la phase isotherme et un pic S2 correspondant à la pyrolyse du kérogène. Selon la nature de l'échantillon et le gradient de température utilisé, il est possible de différencier deux pics S2a et S2b. Ce principe de mesures est décrit dans le document FR-9408383.

Les courbes 32 et 33 correspondent respectivement à la quantité de CO₂ et de CO produit par l'échantillon soumis à la loi de température de la courbe 30. Les courbes 32 et 33 sont fournies par des moyens de mesure en continu du CO₂ et du CO, par exemple des capteurs infrarouges spécifiquement adaptés au présent dispositif. On distingue ici un pic S3, S3' et S3" dont la signification sera explicité ci-après.

La mesure et la lecture en continu pendant la pyrolyse de la teneur en CO₂ permet de bien différencier le pic S3 du pic S3", ce dernier correspondant à du carbone minéral. De plus, la mesure en continu du CO est un complément à la mesure du CO₂, et augmente la précision de calcul et la représentativité des différents index pétroliers.

Sur la figure 3B, la courbe 34 montre la loi de programmation du gradient de température d'oxydation. En effet, selon la présente invention, le four d'oxydation est à température programmable, ce qui permet d'obtenir les pics S4, S5 et S4", en continu et en fonction de la température. La courbe 35 correspond à la mesure du CO₂, la courbe 36 correspond à la mesure en continu du CO.

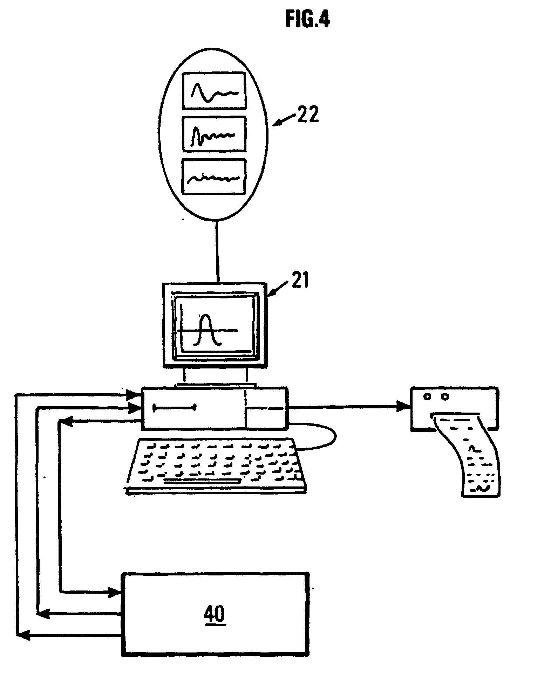

La figure 4 montre l'architecture générale du système de mesure et d'évaluation pétrolière à partir d'un échantillon.

La référence 40 représente l'automate de mesure, les fours et les différents capteurs de signaux, tels que décrits figures 1 et 2. La référence 21 désigne un calculateur, par exemple du type PC, comportant une unité centrale et un écran d'affichage. Le logiciel de pilotage et d'acquisition est du type WINDOWS permettant aisément à l'utilisateur de sélectionner le type d'analyse et le cycle souhaité, et de suivre le déroulement de l'analyse sur un écran. Le tableau 22 symbolise les différents types d'analyse pouvant être mis en oeuvre par le type d'automate selon la présente invention. L'ensemble de mesure permet la possibilité de visualiser et de traiter les résultats pendant l'acquisition de nouvelles mesures. La sortie de courbes ou de tableaux sur imprimante ou sur écran, le traitement partiel des données et l'introduction ou les modifications des paramètres des nouvelles analyses sont un gain de temps énorme pour l'opérateur.

Le calculateur comporte plusieurs logiciels de gestion de plusieurs types d'analyse, par exemple dénommés ci-après sous des noms de marques déposés par l'IFP:
ROCKSIX: acquisition des données Rock-Eval et détermination des températures vraies de pyrolyse.
ROCKINT: interprétation de ces données après correction et entrée d'informations complémentaires.
OPTKIN: détermination des paramètres cinétiques des roches mères.
MATOIL: modèle de maturation et de génération d'hydrocarbures.
GENEX: modèle de génération et d'expulsion d'hydrocarbures par classes de carbone.

On peut définir quatre grands types d'analyses suivant la catégorie des échantillons à traiter (roches brutes, kérogènes et matières organiques pures, échantillons de réservoir ), ou selon les résultats nécessaires et utiles à l'utilisateur (pyrolyses pour la détermination des paramètres cinétiques (OPTKIN), pyrolyses préparatives avec différents paliers et rampe de chauffe, les traitements particuliers, etc...). Le type d'analyse le plus courant est l'analyse Rock-Eval classique qui traite les échantillons de roche suivant les critères définis dans les documents cités en référence. L'analyse de la matière organique concentrée permet d'obtenir le bilan carbone complet de l'échantillon aussi bien en pyrolyse qu'en oxydation. L'analyse dite OPTKIN permet de pyrolyser les échantillons à différentes vitesses pour obtenir des courbes qui seront traitées plus tard par le logiciel de même nom. L'analyse des réservoirs et des tar mats fractionne les composés légers et lourds contenus dans un échantillon de réservoir. L'analyse préparatoire de maturation sert à chauffer un échantillon de roche mère par exemple dans des conditions précises de température (isotherme ou en programmation de température). Au cours de cette chauffe, les produits qui se dégagent sont mesurés. L'échantillon est ensuite traité par pyrolyse et oxydation suivant l'utilisation qui est demandée à ce résidu de la maturation. (OPTKIN ou autres analyses).

Pour chaque type d'analyse, une liste de cycles fixés standards est proposée. Elle pourra être complétée par des cycles particuliers prédéterminés par chaque utilisateur selon ses besoins et ses désirs.

### L'analyse Rock-Eval classique:

Elle est utilisée pour analyser les roches mères et déterminer les paramètres classiques de l'analyse Rock-Eval: quantité d'huile (S1), potentiel pétrolier (S2), quantité de composés oxygénés (S3), Tmax, TOC carbone minéral , IP , HI et OI en vue d'une interprétation ou d'une sélection d'échantillons qui seront soumis à des traitements plus élaborés.

Pour optimiser la durée de l'analyse en fonction des roches à traiter et des renseignements demandés sur les échantillons, l'opérateur choisit dans les différents cycles pré programmés ou fixe de nouveau paramètres.

### L'analyse de la matière organique concentrée et des charbons:

Elle est utilisée pour analyser les kérogènes, les charbons et toutes les autres matières organiques concentrées. La pyrolyse et l'oxydation à haute température donnent le bilan carbone de l'échantillon. Dans cette analyse, la source du carbone présent dans les composés HC, CO₂ et CO est uniquement organique.

La pyrolyse à 750°C donne l'intégralité du pic S2. L'oxydation du résiduel en programmation de température jusqu'à 800°C brûle les cokes entièrement (dans les analyses précédentes, au cours de l'oxydation programmée, l'intégration des courbes CO et CO₂ s'arrête à 650°C et les surfaces obtenues après cette température sont comptabilisées dans la rubrique carbonates).

Suivant le type de matière organique, les conditions de pyrolyse et d'oxydation varient considérablement. Pour optimiser les durées d'analyse et aussi pour permettre un traitement différent des courbes obtenues des cycles différents sont proposés en menu dans le PC.

### L'analyse dite OPTKIN:

Pour déterminer les paramètres cinétiques de la dégradation d'une matière organique, le logiciel OPTKIN a besoin de plusieurs courbes de pyrolyses effectuées à des vitesses différentes avec un facteur d'au moins de 8 entre la vitesse la plus rapide et la vitesse la plus lente. La présente invention permet de choisir les vitesses de programmation dans les limites de 0.2°C/min et 50°C/min. Pour chaque vitesse correspond une température finale qui permet à la courbe de pyrolyse de revenir à sa ligne de base. Par exemple, avec un gradient de 50°C/min, il est nécessaire d'atteindre une température finale de 750°C pour craquer entièrement la matière organique alors qu'à 0.2°C/min le craquage est terminé à 450°C.

On constate qu'avec cette nouvelle possibilité, que les gains de temps seront d'autant plus importants que les vitesses sont plus lentes. (25h gagnées pour la vitesse minimum de 0.2°C/min et 15min pour une vitesse de 5°C/min)

Pour chaque échantillon le logiciel impose au moins deux courbes et au plus dix courbes de pyrolyse dont les vitesses extrêmes sont au minimum dans un rapport 8. Chaque cycle permet donc d'analyser au moins dix fois la même roche avec des vitesses variables. Suivant le cycle utilisé, la température finale est déterminée par la vitesse.

### L'analyse des réservoirs et des Tar-Mats

Il permet de différencier deux pics dans la courbe de pyrolyse: un pic "S2a" correspondant à des hydrocarbures lourds qui n'ont pas été vaporisés pendant l'isotherme et un pic "S2b" qui est le résultat du craquage des résines et des asphaltènes et de la matière organique présente dans l'échantillon. Les résidus après pyrolyse sont oxydés pour déterminer le carbone total.

### L'analyse préparatoire de maturation:

Elle permet de conditionner un échantillon sous gaz inerte à une température déterminée par un isotherme ou en programmation de température. Le résidu est, soit traité immédiatement en pyrolyse et en oxydation, soit récupéré pour une utilisation ultérieure dans une autre analyse selon la présente invention, soit utilisé par un autre appareillage d'une méthode différente.

## Revendications

1. Méthode pour permettre la détermination d'au moins une caractéristique pétrolière d'un échantillon de sédiment géologique, dans laquelle l'échantillon est chauffé en atmosphère non oxydante, sa température étant élevée successivement jusqu'à une première puis une seconde valeur, ladite première valeur étant inférieure à 200°C puis maintenue constante pendant un certain temps, ladite seconde valeur comprise entre 600°C et 850°C étant atteinte selon un gradient de température compris entre 0,2 et 50°C/min à partir de ladite première valeur, on mesure en continu avec un premier détecteur (10) les produits hydrocarbonés dégagés de l'échantillon en cours de chauffe, **caractérisée en ce que** l'on mesure en continu avec un second détecteur, à chaque instant de la période de chauffe dudit échantillon, la quantité de CO2 et la quantité de CO contenues par l'effluent résultant de ladite chauffe.

2. Méthode selon la revendication 1, **caractérisée en ce que** l'on différencie l'oxygène organique de l'oxygène minéral par l'interprétation conjuguée des mesures continues de CO2 et de CO.

3. Méthode selon l'une des revendications 1 ou 2 dans laquelle on place les résidus de ladite chauffe en atmosphère non oxydante dans un autre four où ils sont chauffés en atmosphère oxydante, **caractérisée en ce que** la chauffe en atmosphère oxydante est programmée en température de façon à passer d'une température d'environ 400°C à une température finale d'environ 850°C par un gradient de température compris entre 10 et 30°C/min, **en ce que** l'on mesure en continu, à chaque instant de la période de chauffe desdits résidus, la quantité de CO2 contenu par l'effluent résultant de ladite chauffe, et **en ce que** l'on différencie le CO2 d'origine organique du CO2 d'origine minéral à partir des mesures continues.

4. Méthode selon la revendication 3, **caractérisée en ce que** l'on mesure en continu, à chaque instant de la période de chauffe en atmosphère oxydante desdits résidus, la quantité de CO contenu par l'effluent résultant de ladite chauffe.

5. Méthode selon la revendication 4, **caractérisée en ce que** l'on calcule la quantité de carbone organique total contenue dans ledit échantillon à partir des mesures continues de CO2 et CO obtenues à la suite des cycles de chauffe en atmosphère non oxydante et en atmosphère oxydante.

6. Dispositif pour permettre la détermination d'au moins une caractéristique pétrolière d'un échantillon de sédiment géologique placé dans une nacelle, ledit dispositif comportant un premier moyen de chauffe dudit échantillon sous atmosphère non oxydante, des moyens de mesure de la quantité des produits hydrocarbonés dégagés à la suite de l'introduction de l'échantillon dans ledit premier moyen de chauffe, un second moyen de chauffe sous atmosphère oxydante, des moyens de mesure de la quantité de CO2 contenue dans les effluents évacués des deux moyens de chauffe, **caractérisé en ce que** lesdits moyens de mesure du CO2 comportent une cellule de mesure en continu du CO2 à chaque instant du cycle de chauffe du premier et du second moyen de chauffe et **en ce qu'**il comporte des moyens de mesure en continu de la quantité de CO contenue dans les effluents évacués par les deux moyens de chauffe.

7. Dispositif selon la revendication 6, **caractérisé en ce que** les premier et second moyen de chauffe comportent chacun un four identique et des moyens de programmation de la température de chauffe des deux fours.

8. Dispositif selon la revendication 7, **caractérisé en ce que** lesdits fours comportent un corps cylindrique et une cavité intérieure destinée à recevoir la nacelle contenant l'échantillon, ledit corps ayant trois parties, une partie supérieure comportant un conduit intérieur en communication avec ladite cavité et avec les moyens de mesure de la quantité des produits hydrocarbonés, une partie inférieure comportant l'ouverture de ladite cavité, une partie centrale dans laquelle sera placée la nacelle, **en ce que** les trois parties sont entourées d'un élément électrique de chauffe bobiné hélicoïdalement à pas jointifs pour les deux parties supérieure et inférieure et à pas double pour la partie centrale.

9. Dispositif selon l'une des revendications 6 à 8, **caractérisé en ce qu'**il comporte des moyens d'introduction de la nacelle dans lesdits moyens de chauffe comportant des moyens de mesure de la température au niveau de la nacelle et des moyens de circulation d'un fluide non oxydant, par exemple de l'azote ou de l'hélium, ou d'un fluide oxydant, par exemple de l'air ou de l'oxygène.

10. Dispositif selon l'une des revendications 8 ou 9, **caractérisé en ce que** lesdits moyens de chauffe comportent chacun un moyen de mesure de la température placé dans la paroi de la partie centrale dudit corps, au niveau de la nacelle.

11. Dispositif selon la revendication 10, **caractérisé en ce qu'**il comporte des moyens de régulation et de programmation de la température des moyens de chauffe pilotés par lesdits moyens de mesure de la température desdits moyens d'introduction, et **en ce que** lesdits moyens de régulation et de programmation sont pilotés par lesdits moyens de mesure de la température placés dans la paroi de la partie centrale dudit corps lorsque la nacelle est hors des moyens de chauffe, ceux-ci étant alors ouverts.

12. Dispositif selon la revendication 8, **caractérisé en ce que** ledit moyen de chauffe en atmosphère non oxydante comporte un moyen de balayage de la cavité intérieure du corps de chauffe par un fluide non oxydant lorsque ledit corps est ouvert.

13. Dispositif selon l'une des revendications 6 à 8, **caractérisé en ce qu'**il comporte des moyens de déplacement de la nacelle entre les deux moyens de chauffe et un magasin, comportant un bras équipé d'une pince à nacelle, ledit bras étant commandé en rotation pour déplacer une nacelle vers une des trois positions et en hauteur pour saisir ou déposer la nacelle, et **en ce que** le magasin a la forme d'un plateau rotatif sur lequel différentes nacelles sont disposées en cercle.

14. Dispositif selon la revendication 6, **caractérisé en ce qu'**il comporte un premier couple de cellules de mesure en continu du CO₂ et du CO contenus par l'effluent dégagé par le premier moyen de chauffe et un deuxième couple de cellules de mesure en continu du CO₂ et du CO contenus par l'effluent dégagé par le second moyen de chauffe.

15. Dispositif selon la revendication 6, **caractérisé en ce qu'**il comporte des moyens électroniques d'interface entre les moyens de mesures, de commande de vannes ou de distributeurs, de régulation de la température, de déplacement des nacelles et un calculateur comportant des moyens de mémorisation et d'affichage.

16. Système d'évaluation pétrolière à partir d'échantillon de sédiment géologique, **caractérisé en ce qu'**il comporte:
- un dispositif tel que décrit dans l'une des revendications 6 à 8, 14, 15,
- un calculateur de type PC interfacé avec ledit dispositif et comportant des périphériques de visualisation, d'impression et d'introduction des paramètres de fonctionnement,
ledit calculateur comportant un logiciel multitâche comportant un menu proposant plusieurs cycles préprogrammés d'analyses pouvant être effectués à l'aide dudit dispositif, parmi lesquels: le cycle d'analyse des roches mères, le cycle d'analyse des kérogènes ou charbons, le cycle d'analyse pour déterminer les paramètres cinétiques de la dégradation d'une matière organique, le cycle d'analyse de roches réservoirs, le cycle d'analyse préparative à la maturation.

17. Système selon la revendication 16, **caractérisé en ce que** le logiciel permet l'affichage de mesures ou de courbes caractéristiques en temps réel, ledit logiciel comportant des moyens de modification du cycle choisi en cours d'accomplissement en fonction des mesures ou courbes caractéristiques obtenues.

## Claims

1. A method for determining at least one petroleum characteristic of a geologic sediment sample, wherein the sample is heated in a non-oxidizing atmosphere, the temperature thereof being successively raised to a first value, then to a second value, said first value being below 200°C, then maintained constant for some time, said second value ranging between 600°C and 850°C being reached with a temperature gradient ranging between 0.2°C/min and 50°C/min from said first value, the hydrocarbon-containing products released by the sample during heating are continuously measured by a first detector (10), **characterized in that** the amount of CO₂ and the amount of CO contained in the effluent resulting from said heating are continuously measured by a second detector throughout said sample heating period.

2. A method as claimed in claim 1, **characterized in that** the organic oxygen is differentiated from the inorganic oxygen by combined interpretation of the continuous measurements of CO₂ and CO.

3. A method as claimed in any one of claims 1 or 2, wherein the residues resulting from said heating in a non-oxidizing atmosphere are placed in another oven where they are heated in an oxidizing atmosphere, **characterized in that** the temperature of the oxidizing atmosphere heating is programmed so as to rise from a temperature of about 400°C to an end temperature of about 850°C with a temperature gradient ranging between 10 and 30°C/min, **in that** the amount of CO₂ contained in the effluent resulting from said heating is measured continuously throughout the period of heating of said residues, and **in that** the CO₂ of organic origin is differentiated from the CO₂ of inorganic origin from the continuous measurements.

4. A method as claimed in claim 3, **characterized in that** the amount of CO contained in the effluent resulting from said heating is measured continuously throughout the period of heating of said residues in an oxidizing atmosphere.

5. A method as claimed in claim 4, **characterized in that** the amount of total organic carbon contained in said sample is calculated from the continuous CO₂ and CO measurements obtained after heating cycles in a non-oxidizing atmosphere and in an oxidizing atmosphere.

6. A device for determining at least one petroleum characteristic of a geologic sediment sample placed in a boat, said device including a first means for heating said sample in a non-oxidizing atmosphere, means for measuring the amount of hydrocarbon-containing products released after feeding the sample into said first heating means, a second means for heating in an oxidizing atmosphere, means for measuring the amount of CO₂ contained in the effluents discharged from the two heating means, **characterized in that** said CO₂ measuring means include a cell for measuring continuously the CO₂ throughout the heating cycle of the first and of the second heating means and **in that** it comprises means for continuously measuring the amount of CO contained in the effluents discharged from the two heating means.

7. A device as claimed in claim 6, **characterized in that** the first and the second heating means comprise each an identical oven and means for programming the heating temperature of the two ovens.

8. A device as claimed in claim 7, **characterized in that** said ovens include a cylindrical body and an inner cavity intended to receive the boat containing the sample, said body having three parts, an upper part comprising an inner line communicating with said cavity and with the means for measuring the amount of hydrocarbon-containing products, a lower part comprising the opening of said cavity and a central part in which the boat will be placed, and **in that** the three parts are surrounded by an electric heating element helically wound with contiguous pitches for the upper and the lower parts and with a double pitch for the central part.

9. A device as claimed in any one of claims 6 to 8, **characterized in that** it includes means for feeding the boat into said heating means comprising temperature measuring means at the level of the boat and means for circulation of a non-oxidizing fluid, for example nitrogen or helium, or of an oxidizing fluid, for example air or oxygen.

10. A device as claimed in any one of claims 8 or 9, **characterized in that** said heating means comprise each a temperature measuring means placed in the wall of the central part of said body, at the level of the boat.

11. A device as claimed in claim 10, **characterized in that** it comprises means for regulating and for programming the temperature of the heating means controlled by said temperature measuring means of said feed means, and **in that** said regulation and programming means are controlled by said temperature measuring means placed in the wall of the central part of said body when the boat is outside the heating means, the latter being then open.

12. A device as claimed in claim 8, **characterized in that** said means for heating in a non-oxidizing atmosphere comprises a means for sweeping the inner cavity of the heating body with a non-oxidizing fluid when said body is open.

13. A device as claimed in any one of claims 6 to 8, **characterized in that** it comprises means for moving the boat between the two heating means and a storage support, comprising an arm equipped with a boat clamp, said arm being controlled in rotation in order to move a boat into one of the three positions and in height to seize or to put the boat down, and **in that** the storage support has the shape of a rotary tray on which various boats are arranged in a circle.

14. A device as claimed in claim 6, **characterized in that** it comprises a first pair of cells for measuring continuously the CO₂ and the CO contained in the effluent released by the first heating means and a second pair of cells for measuring continuously the CO₂ and the CO contained in the effluent released by the second heating means.

15. A device as claimed in claim 6, **characterized in that** it comprises electronic interface means between the measuring means, valve or distributor control means, temperature regulation means, boat displacement means and a computer comprising storage and visual display means.

16. A system intended for petroleum evaluation from geologic sediment samples, **characterized in that** it comprises :
- a device as described in any one of claims 6 to 8, 14, 15,
- a PC type computer interfaced with said device and comprising visual display, print and working parameter entry peripherals,
said computer comprising a multi-task software including a menu offering several preprogrammed analysis cycles that can be performed by means of said device, among which : the mother rock analysis cycle, the kerogen or coals analysis cycle, the analysis cycle for determining the kinetic parameters of the degradation of an organic matter, the reservoir rock analysis cycle, the maturation preparative analysis cycle.

17. A system as claimed in claim 16, **characterized in that** the software allows real-time display of characteristic measurements or curves, said software comprising means for changing the selected cycle in progress according to the characteristic measurements or curves obtained.

## Patentansprüche

1. Verfahren zur Bestimmung wenigstens einer Erdölcharakteristik einer geologischen Sedimentprobe, bei der die Probe in nicht-oxidierender Atmosphäre erwärmt, ihre Temperatur sukzessive bis auf einen ersten, dann einen zweiten Wert erwärmt wird, dieser erste Wert unterhalb 200°C liegt, dann während eines gewissen Zeitraums konstant gehalten wird, wobei dieser zweite Wert zwischen 600°C und 850°C gemäß einem Temperaturgradienten zwischen 0,2 und 50°C/min, ausgehend von dem ersten Wert, erreicht wird, man kontinuierlich mit einem ersten Detektor (10) die kohlenwasserstoffhaltigen Produkte misst, die aus der Probe während der Erwärmung freigesetzt werden, **dadurch gekennzeichnet, dass** man kontinuierlich mit einem zweiten Detektor bei jedem Augenblick der Heizperiode dieser Probe die CO2-Menge und die CO-Menge, die in dem aus dieser Erwärmung resultierenden Abstrom enthalten sind, misst.

2. Verfahren nach Anspruch 1, **dadurch gekennzeichnet, dass** man den organischen Sauerstoff vom mineralischen Sauerstoff durch konjugierte Interpretation der kontinuierlichen Messungen von CO2 und CO differenziert.

3. Verfahren nach einem der Ansprüche 1 oder 2, bei dem man die Rückstände dieser Erwärmung in nicht-oxidierender Atmosphäre in einem anderen Ofen einsetzt, wo sie in oxidierender Atmosphäre erwärmt werden, **dadurch gekennzeichnet, dass** die Erwärmung in oxidierender Atmosphäre temperaturprogrammiert derart wirkt, dass von einer Temperatur von etwa 400°C zu einer Endtemperatur von etwa 850°C gemäß eines Temperaturgradienten übergegangen wird, der zwischen 10 und 30°C/min liegt und dass man kontinuierlich in jedem Augenblick der Heizperiode dieser Rückstände, die Menge an CO2 misst, die durch den aus der Erwärmung resultierenden Abstrom enthalten ist und dass man das organische Ursprungs-CO2 vom mineralischen Ursprungs-CO2, ausgehend von den kontinuierlichen Messungen differenziert bzw. unterscheidet.

4. Verfahren nach Anspruch 3, **dadurch gekennzeichnet, dass** man kontinuierlich in jedem Augenblick der Heizperiode in oxidierender Atmosphäre diese Rückstände, die CO-Menge misst, die in dem aus dieser Erwärmung resultierenden Abstrom enthalten sind.

5. Verfahren nach Anspruch 4, **dadurch gekennzeichnet, dass** man die Gesamtmenge an organischem Kohlenstoff, die in dieser Probe enthalten ist, ausgehend von kontinuierlichen Messungen von CO2 und CO misst, die anschließend an Heizzyklen in nicht-oxidierender Atmosphäre und in oxidierender Atmosphäre erhalten wurden.

6. Vorrichtung zur Bestimmung wenigstens einer Erdölcharakteristik einer geologischen in einem Schiffchen eingesetzten Sedimentprobe, wobei die Vorrichtung ein erstes Mittel zum Erwärmen dieser Probe unter nicht oxidierender Atmosphäre, Messmittel für die Menge der kohlenwasserstoffhaltigen, anschließend an die Einführung der Probe in dieses erste Heizmittel freigesetzten Produkte, ein zweites Heizmittel in nicht oxidierender Atmosphäre, Messmittel für die CO2-Menge, die in den Abströmen enthalten ist, die aus den beiden Heizmitteln abgezogen werden, umfasst, **dadurch gekennzeichnet, dass** diese Messmittel für das CO2 eine Zelle zur kontinuierlichen Messung des CO2 in jedem Heizaugenblick des ersten und zweiten Heizmittels umfassen und dass die Vorrichtung Mittel zur kontinuierlichen Messung der CO-Menge, die in den durch die Heizmittel abgezogenen Abströmen enthalten ist, umfasst.

7. Vorrichtung nach Anspruch 6, **dadurch gekennzeichnet, dass** die ersten und zweiten Heizmittel je einen identischen Ofen und Mittel zur Programmierung der Heiztemperatur der beiden Öfen umfassen.

8. Vorrichtung nach Anspruch 7, **dadurch gekennzeichnet, dass** diese Öfen einen zylindrischen Körper und einen Innenhohlraum aufweisen, der zur Aufnahme des die Probe enthaltenden Schiffchens bestimmt ist, wobei dieser Körper drei Teile, einen oberen Teil mit einer Innenleitung in Verbindung mit diesem Hohlraum und mit diesen Messmitteln für die Menge der kohlenwasserstoffhaltigen Produkte, einen unteren Teil mit der Öffnung dieses Hohlraums und einen mittigen Teil aufweist, in dem das Schiffchen angeordnet wird und dass die drei Teile von einem elektrischen Heizelement umschlossen sind, das spiralartig mit aneinander angrenzenden Windungen für die beiden oberen und unteren Teile und mit Doppelwindung für den mittigen Teil umwickelt ist.

9. Vorrichtung nach einem der Ansprüche 6 bis 8, **dadurch gekennzeichnet, dass** sie Mittel zum Einführen des Schiffchens in diese Heizmittel mit Messmitteln für die Temperatur in Höhe des Schiffchens und Mittel zur Zirkulation eines nicht oxidierenden Fluids, beispielsweise von Stickstoff oder Helium oder eines oxidierenden Fluids, beispielsweise Luft oder Sauerstoff, umfasst.

10. Vorrichtung nach einem der Ansprüche 8 oder 9, **dadurch gekennzeichnet, dass** diese Heizmittel je ein Mittel zur Messung der Temperatur, angeordnet in der Wandung des zentralen Teils dieses Körpers oder Gehäuses in Höhe des Schiffchens umfassen.

11. Vorrichtung nach Anspruch 10, **dadurch gekennzeichnet, dass** sie Mittel zur Steuerung und Programmierung der Temperatur der Heizmittel, hilfsgesteuert durch die Messmittel der Temperatur für diese Einführungsmittel, umfasst und dass diese Steuer- und Programmierungsmittel durch diese Messmittel für die Temperatur (hilfs)gesteuert sind, die in der Wandung des mittigen Teils dieses Gehäuses angeordnet sind, wenn das Schiffchen sich außerhalb der Heizmittel befindet, wobei diese dann offen sind.

12. Vorrichtung nach Anspruch 8, **dadurch gekennzeichnet, dass** dieses Heizmittel in nicht-oxidierender Atmosphäre ein Mittel zum Spülen des Innenhohlraums des Heizkörpers durch ein nicht oxidierendes Fluid, wenn dieser Körper offen ist, umfasst.

13. Vorrichtung nach einem der Ansprüche 6 bis, 8, **dadurch gekennzeichnet, dass** sie Mittel zur Bewegung oder Verschiebung des Schiffchens zwischen den beiden Heizmitteln und einem Speicher umfasst, der einen mit einer Schiffchenzange ausgestatteten Arm umfasst, wobei der Arm hinsichtlich seiner Drehbewegung gesteuert ist, um ein Schiffchen gegen eine der drei Stellungen und in der Höhe zu verschieben, um das Schiffchen zu erfassen oder abzusetzen, und dass der Speicher die Form einer Drehscheibe hat, auf der unterschiedliche Schiffchen kreisförmig angeordnet sind.

14. Vorrichtung nach Anspruch 6, **dadurch gekennzeichnet, dass** sie ein erstes Paar von kontinuierlichen Messzellen des CO2 und CO, die vom Abstrom gefüllt sind, umfasst, der von dem ersten Heizmittel freigegeben ist und ein zweites Paar von Zellen zum kontinuierlichen Messen des CO2 und des CO umfasst, die vom Abstrom gefüllt sind, der vom zweiten Heizmittel freigegeben wurde.

15. Vorrichtung nach Anspruch 6, **dadurch gekennzeichnet, dass** sie elektronische Interface-Mittel zwischen den Messmitteln, den Steuermitteln für die Ventile oder die Verteiler, der Steuerung der Temperatur, der Verschiebung der Schiffchen und einen Rechner umfasst, der Speicher- und Anzeigemittel aufweist.

16. System zur Bewertung von Erdöl, ausgehend von einer geologischen Sedimentprobe, **dadurch gekennzeichnet, dass** es umfasst:
- eine Vorrichtung wie in den Ansprüchen 6 bis 8, 14, 15 beschrieben,
- einen Rechner vom Typ PC mit Interface bezüglich der Vorrichtung und mit peripheren Sichtbarmachungs-, Druck- und Einführungsmitteln für Betriebsparameter, wobei dieser Rechner Vielzweck-Software aufweist, die ein Menü umfasst, weches mehrere vorprogrammierte Analysezyklen vorschlägt, die mit Hilfe dieser Vorrichtung ausgeführt werden können, unter denen: der Zyklus der Analyse der Muttergesteine, der Zyklus der Analyse der Kerogene oder Kohlen, der Zyklus der Analyse zur Bestimmung der kinetischen Parameter des Abbaus eines organischen Materials, der Zyklus der Analyse der Speichergesteine und der Zyklus der präparativen Analyse vor der Reifung zu nennen sind.

17. System nach Anspruch 16, **dadurch gekennzeichnet, dass** die Software die Anzeige von Messungen oder charakteristischen Kurven in Realzeit ermöglicht, wobei die Software Mittel zur Modifikation des Zyklus umfasst, der im Verlauf zur Erreichung des Ziels als Funktion der Messungen oder erhaltenen Charakteristiken gewählt wurde.
